# EUROPÄISCHE PATENTANMELDUNG

(11) **EP 1 099 697 A1**
(43) Veröffentlichungstag der Anmeldung: **16.05.2001**
(21) Anmeldenummer: 00121999.7
(22) Anmeldetag: 10.10.2000
(51) Int. Cl.: C07D 307/62

(54) **Verfahren zur Herstellung von Alkalisalzen der L-Ascorbinsäure**

(30) Priorität: 12.11.1999 DE 19954511
(71) Anmelder: BASF AKTIENGESELLSCHAFT, 67056 Ludwigshafen (DE)
(72) Erfinder: Burst, Wolfram, 68199 Mannheim (DE); Kaibel, Gerd, Dr., 68623 Lampertheim (DE); Böttcher, Andreas, Dr., 69226 Nussloch (DE); Kessler, Veronique, 67063 Ludwigshafen (DE); Kuntze, Thomas, Dr., 67459 Böhl-Iggelheim (DE)

(57) **Zusammenfassung**

Die Erfindung betrifft ein Verfahren zur Herstellung von Alkalisalzen der L-Ascorbinsäure, enthaltend die folgenden Schritte:
a) Veresterung von 2-Keto-L-gulonsäure oder Diaceton-2-keto-L-gulonsäure mit einem C₁-C₁₀-Alkohol in Gegenwart eines sauren Katalysators,
b) Umlagerung des gebildeten 2-Keto-L-gulonsäure-C₁-C₁₀-alkylesters in Gegenwart eines C₁-C₁₀-Alkalialkoholats,
dadurch gekennzeichnet, daß man die Verfahrensschritte a) und b) jeweils kontinuierlich durchführt.

## Beschreibung

Die vorliegende Erfindung betrifft ein kontinuierliches Verfahren zur Herstellung von Alkalisalzen der L-Ascorbinsäure durch Veresterung von 2-Keto-L-gulonsäure oder Diaceton-2-keto-L-gulon-säure mit einem C₁-C₁₀-Alkohol in Gegenwart eines sauren Katalysators und Umlagerung des gebildeten 2-Keto-L-gulonsäure-C₁-C₁₀-alkylesters in Gegenwart eines C₁-C₁₀-Alkalialkoholats.

Zur Herstellung von L-Ascorbinsäure sind in der Vergangenheit eine Vielzahl von Verfahrensvarianten beschrieben worden. Eine Übersicht findet sich u.a. in Crawford et al., Adv. Carbohydrate Chem. 37, 79 (1980) sowie in Ullmann's Encyclopedia of Industrial Chemistry, Vol. A27, 551-557 (1996).

Prinzipiell haben sich zwei Verfahrensvarianten für die großtechnische Synthese von Vitamin C etabliert - eine sauer und eine basisch katalysierte Umlagerung von 2-Keto-L-gulonsäure zu L-Ascorbinsäure bzw. zu L-Ascorbaten.

Die sauer katalysierte Lactonisierung von 2-Keto-L-gulonsäure ist zwar ein einfacher Batch-Prozeß, aber im Umfeld ist ein hoher apparativer Aufwand zur Katalysatorabtrennung und Isolierung des Wertproduktes erforderlich. Die basisch katalysierte Lactonisierung ist ebenfalls ein klassischer Mehrstufenprozeß, der die Herstellung eines Esters der 2-Keto-L-gulonsäure, die Lactonisierung zum Ascorbinsäure-Salz und Freisetzen der Ascorbinsäure unter sauren Bedingungen umfaßt.

Für beide Syntheserouten sind eine Vielzahl von Patenten bzw. Patentanmeldungen bekannt.

So ist in der US 2,185,383 die Umsetzung von 2-Keto-L-gulonsäure mit konzentrierter Salzsäure und Essigsäure als Lösungsmittel beschrieben.

JP-OS 58-177986 beschreibt ein Verfahren, das die Zugabe von Ethanol und Aceton zu dem Natrium-Salz der 2-Keto-L-gulonsäure, die Neutralisation mit Salzsäure, die Abtrennung des ausgefällten Natriumchlorids durch Filtration und anschließend das Halten der Reaktionsmischung bei Temperaturen im Bereich von 25°C bis 75°C umfaßt, wodurch L-Ascorbinsäure erhalten wird.

In der JP-AS 48-15931 wird die Umsetzung von 2-Keto-L-gulonsäure mit einer Mineralsäure in einem inerten Lösungsmittel in Gegenwart einer oberflächenaktiven Substanz beschrieben.

WO 87/00839 beansprucht ein Verfahren, bei dem eine Aufschlämmung von 2-Keto-L-gulonsäure in einem inerten organischen Lösungsmittel in Gegenwart eines grenzflächenaktiven Mittels unter Säurekatalyse zu L-Ascorbinsäure umgesetzt wird.

DE-A-195 47 073 beschreibt ein Verfahren zur Herstellung von L-Ascorbinsäure durch Umsetzung von 2-Keto-L-gulonsäure mit wäßriger Mineralsäure in einem Lösungsmittelgemisch, enthaltend ein inertes organisches Lösungsmittel, ein aliphatisches Keton sowie ein Säurechlorid.

WO 99/07691 beschreibt die Umsetzung von 2-Keto-L-gulonsäure mit konzentrierter Salzsäure bei Temperaturen zwischen 40 und 80°C.

EP-A-0 671 405 offenbart ein Verfahren zur Herstellung von 2-Keto-L-gulonsäure-methyl- oder -ethylester durch Veresterung von 2-Keto-L-gulonsäure mit Methanol bzw. Ethanol in Gegenwart eines sauren Ionenaustauschers. Ferner ist in dieser Anmeldeschrift zu lesen, daß die o.g. Ester einer alkalischen Umlagerung (Lactonisierung) zur Ascorbinsäure oder zu einem Salz davon unterworfen werden können.

US 5,391,770 beschreibt die Veresterung von 2-Keto-L-gulonsäure mit anschließender basenkatalysierter Lactonisierung der gebildeten Ester zu Salzen der L-Ascorbinsäure und Freisetzung der Ascorbinsäure durch Zugabe einer starken Säure.

In der japanischen Auslegeschrift 22 113/75 wird die Veresterung von 2-Keto-L-gulonsäure mit Butanol und die anschließende säurekatalysierte Lactonisierung in Benzol als Lösungsmittel beschrieben.

Die sauer katalysierte Lactonisierung erfordert in der Regel lange Reaktionszeiten und demzufolge große Apparatevolumina, den Einsatz eines inerten Lösungsmittels und eine aufwendige Katalysatorabtrennung.

Günstigere Raum-Zeit-Ausbeuten werden bei der basisch katalysierten Lactonisierung durch schnellere Umlagerung des Esters erzielt. Bei dieser Syntheseroute ist jedoch auf eine vollständige Veresterung der 2-Keto-L-gulonsäure und auf die Einhaltung wasserfreier Reaktionsbedingungen zur Vermeidung von Verseifungsreaktionen zu achten. Dies gelingt in der Regel trotz Einsatz einer Rührkesselkaskade und aufwendiger Entwässerung des Veresterungsalkohols nicht immer zufriedenstellend.

Die basisch katalysierte Lactonisierung z.B. mit NaHCO₃ oder NaOH in Methanol, liefert in der Regel nicht umgesetzte 2-Keto-L-gulonsäure als unerwünschtes Nebenprodukt im Natriumascorbat. Weiterhin führt bei Einsatz von z.B. Natronlauge in Methanol der hohe Wasseranteil zu unerwünschten gefärbten Nebenprodukten und zu einer Verminderung der Ausbeute durch die gute Löslichkeit des Natriumascorbats in Wasser.

Bisher sind nur kontinuierliche Verfahren zur Veresterung der 2-Keto-L-gulonsäure beschrieben worden (siehe EP-A-0 671 405). Dagegen ist die Lactonisierung nach dem Stand der Technik bis dato ein klassischer diskontinuierlicher Prozeß.

Es war daher Aufgabe der vorliegenden Erfindung, ein Verfahren zur Herstellung von Alkalisalzen der L-Ascorbinsäure bereitzustellen, das die oben genannten Nachteile nicht aufweist.

Diese Aufgabe wurde gelöst durch ein Verfahren zur Herstellung von Alkalisalzen der L-Ascorbinsäure, enthaltend die folgenden Schritte:
a) Veresterung von 2-Keto-L-gulonsäure oder Diaceton-2-keto-L-gulonsäure mit einem C₁-C₁₀-Alkohol in Gegenwart eines sauren Katalysators,
b) Umlagerung des gebildeten 2-Keto-L-gulonsäure-C₁-C₁₀-alkyl-esters in Gegenwart eines C₁-C₁₀-Alkalialkoholats,
dadurch gekennzeichnet, daß man die Verfahrensschritte a) und b) jeweils kontinuierlich durchführt.

Als Alkalisalze der L-Ascorbinsäure sind bevorzugt Natrium-, Kalium- und Lithiumsalze, besonders bevorzugt Natriumsalze gemeint.

Zur Veresterung von 2-Keto-L-gulonsäure oder Diaceton-2-keto-L-gulonsäure im Verfahrensschritt a) eignen sich prinzipiell alle C₁-C₁₀-Alkohole, vorteilhafterweise gesättigte, verzweigte oder unverzweigte Alkylalkohole mit einer Kohlenstoffanzahl größer gleich 3, bevorzugt Alkohole mit einem Alkylrest von 3 bis 10 Kohlenstoffatomen, beispielsweise n-Propanol, Isopropanol, 1-Butanol, 2-Butanol, 2-Methyl-1-propanol, 2-Methyl-2-propanol, 1-Pentanol, 2-Pentanol, 3-Pentanol, 1-Hexanol, 2-Hexanol, 1-Heptanol, 2-Heptanol, 1-Octanol, 2-Octanol, 3-Octanol, 1-Nonanol, 2-Nonanol, 1-Decanol, 2-Decanol, 4-Decanol, besonders bevorzugt C₃-C₈-Alkohole, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol. Ganz besonders bevorzugte Alkohole sind n-Butanol und n-Pentanol.

Der Alkohol wird dabei in einem 1- bis 10-fachen, bevorzugt 2-bis 8-fachen, besonders bevorzugt 3- bis 6-fachen molaren Über-schuß, bezogen auf die eingesetzte 2-Keto-L-gulonsäure bzw. Diaceton-2-keto-L-gulonsäure eingesetzt.

Im Verlauf der Veresterungsreaktion kann sowohl das als Lösungsmittel mitgeführte als auch das bei der Veresterung zusätzlich gebildete Wasser als leichter siedendes Azeotrop aus dem Reaktionsraum über die Gasphase entfernt werden.

Falls erforderlich kann nach der Kondensation eine Phasentrennung (Alkohol/Wasser) mit Rückführung des Veresterungsalkohols erfolgen. In der Regel ist dabei eine vollständige Entwässerung des rückgeführten Alkohols, z.B. durch Membranverfahren oder durch Destillation, nicht erforderlich, da erfindungsgemäß eine vollständige Entwässerung im Reaktionsraum, beispielsweise im Gegenstromverfahren erfolgt.

Bei der Veresterung von 2-Keto-L-gulonsäure mit C₁-C₃-Alkoholen kann es aufgrund der niedrigen Siedepunkte dieser Alkohole bei der destillativen Wasserabtrennung zu unerwünschten Verlusten an Alkoholen und damit zu Ausbeuteverlusten kommen. Durch entsprechende Ergänzung der Alkoholverluste während der Reaktion läßt sich die Veresterungsrate wieder steigern.

Durch Zusatz eines sauren Katalysators wird die Veresterungsreaktion in an sich bekannter Weise katalysiert. Der Katalysator wird dabei in Mengen von 0,001 bis 0,2 Mol, bevorzugt in Mengen von 0,005 bis 0,1 Mol, besonders bevorzugt 0,005 bis 0,05 Mol pro Mol 2-Keto-L-gulonsäure bzw. Diaceton-2-keto-L-gulonsäure eingesetzt.

Als Veresterungskatalysatoren können in der Regel alle an sich bekannten homogenen oder heterogenen sauren Katalysatoren eingesetzt werden.

Bevorzugt wird die Veresterung in Gegenwart eines sauren Homogen-oder Heterogenkatalysators ausgewählt aus der Gruppe, bestehend aus Mineralsäuren, organischen Sulfonsäuren, organischen Carbonsäuren, sauren Ionenaustauscherharzen und Festbettkatalysatoren mit sauren Reaktionszentren wie z.B. sauren Zeolithen durchgeführt.

Als homogene Katalysatoren sind beispielsweise Mineralsäuren bzw. deren Ester geeignet. Dazu zählen insbesondere Phosphorsäure, Phosphorsäure-monobutylester, Phosphorsäure-dibutylester, Phosphorsäure-monopentylester, Phosphorsäure-dipentylester, Schwefelsäure, Schwefelsäure-monobutylester, Schwefelsäure-monopentyl-ester, Chlorwasserstoff. Bevorzugte organische Sulfonsäuren sind p-Toluolsulfonsäure, Methansulfonsäure, Trifluormethansulfonsäure und Chlorsulfonsäure. Von den organischen Carbonsäuren lassen sich Trifluoressigsäure aber auch 2-Keto-L-gulonsäure, Diaceton-2-keto-L-gulonsäure oder Ascorbinsäure als Veresterungskatalysatoren einsetzen.

Besonders bevorzugt verwendet man Schwefelsäure, p-Toluolsulfonsäure, Methansulfonsäure oder Monoalkylsulfate der verwendeten Alkohole. Die Monoalkylsulfate spalten bei Temperaturen oberhalb von 70°C Schwefelsäure ab [Popelier, Bull. Soc. Chim. Belg. 35, 265 (1926)], die dann katalytisch wirkt. Ganz besonders bevorzugte homogene saure Katalysatoren sind Schwefelsäure und p-Toluolsulfonsäure.

Im Falle der o.g. homogenen Katalysatoren können diese entweder einem der Edukte vor der Reaktion zugemischt werden oder als separater Strom in den Reaktor eingespeist werden.

Heterogene Katalysatoren sind vorteilhafterweise im Reaktor in der heißen Reaktionszone fixiert. Für den Einbau heterogener Katalysatoren in Destillationskolonnen sind in der Literatur zahlreiche Konstruktionsmöglichkeiten beschrieben. Hierzu gehören Verweilzeitböden, bei denen der Katalysator auf den Böden oder in deren Ablaufschächten angeordnet sein kann, ferner beschichtete Füllkörper oder beschichtete Packungen beispielsweise Keramikpakkungen, gewickelte und strukturierte Packungen mit eingewebtem Katalysator.

Als heterogene Katalysatoren kommen die an sich bekannten sauren Katalysatoren, bevorzugt saure Kationentauscher als auch Zeolithe, in Frage.

Unter der Bezeichnung "saure Kationenaustauscher" sind kommerziell erhältliche Harze bzw. Detoxane zu verstehen, wie z.B. Lewatit® S 100, SP 112 oder Lewatit® 2631 (Bayer) oder Amberlite® 18 und IRA 120 oder Amberlyst® 15 oder Duolite® C 20, C 26 und C 264 (Rohm & Haas) oder Dowex® Ionentauscher.

Zeolithe sind kristalline Aluminosilikate, die eine hochgeordnete Struktur mit einem starren dreidimensionalen Netzwerk von SiO₄-oder AlO₄-Tetraedern besitzen, die durch gemeinsame Sauerstoff-atome verbunden sind. Das Verhältnis der Si- und Al-Atome zu Sauerstoff beträgt 1:2. Die Elektrovalenz der Aluminium enthaltenden Tetraeder ist durch Einschluß von Kationen in den Kristall, z.B. eines Alkali- oder Wasserstoffatoms ausgeglichen. Ein Kationenaustausch ist daher möglich. Die Räume zwischen den Tetraedern sind vor der Dehydration durch Trocknen bzw. Calcinieren von Wassermolekülen besetzt.

Geeignete Zeolithe sind z.B. solche vom Pentasil-Typ, besonders Aluminosilikat-Zeolithe oder Borosilikat-Zeolithe. Durch partielle Verkokung (pre-coke) ist es möglich, die Aktivität des Katalysators für ein Selektivitätsoptimum des gewünschten Reaktionsproduktes einzustellen.

Die Veresterung wird erfindungsgemäß bei einer Temperatur im Bereich von 40 bis 250°C, bevorzugt von 70 bis 200°C, besonders bevorzugt im Bereich von 80 bis 150°C durchgeführt.

Die Verweilzeit des Reaktionsgemisches im Reaktionsraum liegt im Bereich von 1 bis 2000 Sekunden, bevorzugt 20 bis 300 Sekunden, besonders bevorzugt im Bereich von 30 bis 250 Sekunden.

Die Veresterung erfolgt erfindungsgemäß im Druckbereich zwischen 1 und 2000 mbar und wird bevorzugt zwischen 150 und 1000 mbar, besonders bevorzugt zwischen 200 und 950 mbar ausgeführt.

Neben 2-Keto-L-gulonsäure kann auch Diaceton-2-keto-L-gulonsäure unter den oben genannten Bedingungen in gleicher Weise verestert werden. Dabei erfolgt zusätzlich eine Abspaltung der AcetonSchutzgruppen. Für die Abspaltung werden zwei Moläquivalente Wasser benötigt, während gleichzeitig ein Moläquivalent Wasser bei der Veresterungsreaktion gebildet wird. In einfachster Weise setzt man daher das Monohydrat der Diaceton-2-keto-L-gulonsäure ein. Die Reaktion erfolgt im gleichen, wie oben beschriebenen Druck- und Temperaturbereich. Das gebildete Aceton wird als Leichtsieder während der Veresterungsreaktion zu Beginn bzw. zusammen mit überschüssigem, wasserhaltigem Lösungsmittel abdestilliert und kann nach Isolierung und Reingewinnung zurückgeführt und beispielsweise zur Synthese von Diaceton-2-keto-L-gulonsäure nach dem klassischen Reichstein-Verfahren wiederverwendet werden. Bei Einsatz von wasserfreier Diaceton-2-keto-L-gulonsäure muß zusätzlich 1 mol Wasser zugesetzt werden.

Für das erfindungsgemäße Verfahren wird bevorzugt 2-Keto-L-gulonsäure als Ausgangsmaterial eingesetzt. Die Säure kann dabei sowohl in kristalliner Form, beispielsweise als getrocknetes oder schleuderfeuchtes Monohydrat, als wasserfreie Verbindung als auch als wäßrige Lösung, beispielsweise als aufkonzentrierte Fermentationslösung eingesetzt werden.

Das Monohydrat der 2-Keto-L-gulonsäure fällt in der Regel bei der Kristallisation aus Wasser oder wasserhaltigen, organischen Lösungsmitteln an. Durch Abschleudern der Kristallmaische ist feuchtes Monohydrat zugänglich. Dieses kann als schleuderfeuchtes Produkt direkt in der erfindungsgemäßen Veresterungsreaktion eingesetzt oder unter milden Bedingungen getrocknet werden.

Vorteilhafterweise kann man bei dem erfindungsgemäßen Verfahren auf die Trocknung bzw. Entwässerung des Monohydrates der 2-Keto-L-gulonsäure verzichten, da bei der nachfolgenden, erfindungsgemäßen Aktivierungsreaktion ohnehin eine azeotrope Entwässerung durchgeführt wird.

Der Umsatzgrad der 2-Keto-L-gulonsäure bei der Veresterungsreaktion liegt im erfindungsgemäßen Verfahren deutlich über 90%, bevorzugt über 95%, besonders bevorzugt in einem Bereich von 97 bis 99,9%.

Der im Verfahrensschritt a) gebildete 2-Keto-L-gulonsäure-C₁-C₁₀-alkylester wird anschließend im Schritt b) in Gegenwart eines C₁-C₁₀-Alkalialkoholats kontinuierlich in das entsprechende Alkalisalz der L-Ascorbinsäure überführt.

Die für die Alkalialkoholate verwendeten Alkohole sind die bereits eingangs genannten C₁-C₁₀-, bevorzugt C₃-C₁₀-, besonders bevorzugt C₃-C₈-Alkohole, insbesondere solche Verbindungen, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol.

Die Lactonisierung im Verfahrensschritt b) wird erfindungsgemäß bei einer Temperatur im Bereich von 40 bis 200°C, bevorzugt von 60 bis 150°C, besonders bevorzugt im Bereich von 80 bis 120°C durchgeführt.

Die Verweilzeit des Reaktionsgemisches im Reaktor liegt im Bereich von 20 Sekunden bis 60 Minuten, bevorzugt 30 Sekunden bis 10 Minuten, besonders bevorzugt im Bereich von 40 Sekunden bis 3 Minuten.

Die Reaktion in der Stufe b) erfolgt erfindungsgemäß im Druckbereich zwischen 1 und 4000 mbar und wird bevorzugt zwischen 150 und 1000 mbar, besonders bevorzugt zwischen 200 und 950 mbar ausgeführt.

Eine besondere Ausführungsform des erfindungsgemäßen Verfahrens zeichnet sich dadurch aus, daß man in einem zusätzlichen Verfahrensschritt c) das C₁-C₁₀-Alkalialkoholat in-situ erzeugt, beispielsweise durch Umsetzung des entsprechenden C₁-C₁₀-Alkohols mit einer wäßrigen Alkalihydroxidlösung.

Verfahrensschritt c) wird erfindungsgemäß bei einer Temperatur im Bereich von 40 bis 250°C, bevorzugt von 70 bis 200°C, besonders bevorzugt im Bereich von 80 bis 150°C durchgeführt.

Die Verweilzeit liegt im Bereich von 1 bis 2000 Sekunden, bevorzugt 20 bis 300 Sekunden, besonders bevorzugt im Bereich von 30 bis 250 Sekunden.

Die Bildung des Alkoholats erfolgt erfindungsgemäß im Druckbereich zwischen 1 und 4000 mbar und wird bevorzugt zwischen 150 und 1000 mbar, besonders bevorzugt zwischen 200 und 950 mbar ausgeführt.

Mögliche Apparaturen zur Durchführung des erfindungsgemäßen kontinuierlichen Verfahrens sind u.a. Fallfilm- oder Fallstromverdampfer, Sambay- und Luwa-Dünnschichtverdampfer [= Verdampfer, bei denen mittels rotierender Wischblätter oder Rollen dünne Flüssigkeitsfilme erzeugt werden], Rotationsverdampfer aber auch Dünnschicht-Rektifikationskolonnen [z.B. Füllkörper- und Filmkolonnen, Rektifikatoren mit rotierenden Einsätzen (Sprühkolonnen)]. Eine nähere Beschreibung der hier genannten Reaktoren findet sich in Ullmanns Encyklopädie der technischen Chemie, 4. Auflage, Band 2 (1972), S. 516, 533-537, 652-660 sowie in Band 3 (1973), S. 386-388.

Als bevorzugte Reaktoren sind Dünnschichtverdampfer oder Fallstromverdampfer sowie besonders bevorzugt die u.a. in Chem. Ing. Techn. 43, 1101 (1971) beschriebenen Reaktionskolonnen mit Abtriebs- und Verstärkungsteil zu nennen.

Die Konstruktion der Reaktionskolonnen kann dabei eine Anordnung von thermisch gekoppelten Destillationskolonnen oder bevorzugt sogenannte Trennwandkolonnen darstellen.

Die im Rahmen des erfindungsgemäßen Verfahrens bevorzugt verwendete Reaktivdestillation ist literaturbekannt und für homogen und heterogen katalysierte Reaktionen oder autokatalytisch ablaufende Reaktionen u.a. beschrieben in Chem. Ing. Tech. 50 (1978) 8, 586-592, Chem. Eng. 10 (1998), 158-163 und Chem. Tech., 5 (1997), 37-45 sowie in EP-A-0 454 719.

Bei homogen katalysierten Reaktionen werden meist nichtflüchtige oder hochsiedende Katalysatoren eingesetzt, wie z.B. Schwefelsäure, p-Toluolsulfonsäure, Laugen oder Alkoholate. In Einzelfällen können jedoch auch homogene Katalysatoren verwendet werden, deren Siedepunkt im Bereich der Reaktanden liegt, beispielsweise Salpetersäure, die durch die Destillationswirkung überwiegend in der Kolonne verbleiben.

Heterogene Katalysatoren werden meist in Form von festen Katalysatorbetten innerhalb der Kolonne oder in separaten Behältern außerhalb der Kolonne untergebracht. Sie können als Schüttungen zum Einsatz kommen, beispielsweise bei Ionentauscherharzen, zu Füllkörpern geformt werden, beispielsweise zu Raschigringen gepreßte Ionentauscher, in Filtergewebe eingebracht und zu Rollen (sog. Bales) oder Kolonnenpackungen geformt werden, auf Destillationspackungen aufgebracht werden oder als Suspension in der Kolonne eingesetzt werden.

Eine besondere Ausführungsform der Reaktionskolonnen stellt die sogenannte Trennwandkolonne dar. Dieser Kolonnentyp ist beispielsweise beschrieben in Chem. Eng. Technol. 10 (1987) 92-98, Process Engineering 2 (1993) 33-34 und Chemical Engineering 7 (1997) 72-76.

Eine Beschreibung von thermisch gekoppelten Destillationskolonnen, die in verschiedener apparativer Ausgestaltung ausgeführt sein können findet sich ebenfalls in den oben genannten Stellen in der Fachliteratur. Trennwandkolonnen und thermisch gekoppelte Kolonnen bieten gegenüber der Anordnung von konventionellen Destillationskolonnen sowohl hinsichtlich des Energiebedarfs als auch der Investitionskosten Vorteile und werden daher zunehmend industriell eingesetzt.

Trennwandkolonnen und thermisch gekoppelte Kolonnen können sowohl als Packungskolonnen mit Füllkörpern oder geordneten Packungen oder als Bodenkolonnen ausgeführt werden.

EP-A-0 126 288 beschreibt eine Trennwandkolonne, in der chemische Reaktionen durchgeführt werden. Durch eine definierte Zugabe von homogenen Katalysatoren hinsichtlich der Zugabestelle und ihrer relativen Flüchtigkeit zu den einzelnen Reaktanten lassen sich chemische Reaktionen gezielt auf bestimmte Teilbereiche des Kolonnensystems eingrenzen. Hinsichtlich des Einsatzes heterogener Katalysatoren wird beschrieben, daß sie in Form von Ionentauschern in Verweilzeitbehältern außerhalb des Kolonnenkörpers verwendet werden können.

Das erfindungsgemäße Verfahren ist ferner dadurch gekennzeichnet, daß man mindestens einen der o.g. Verfahrensschritte a) bis c) in einer Destillationskolonne, insbesondere in einer Trennwandkolonne durchführt.

Bevorzugt werden alle drei Verfahrensschritte a) bis c) in einer gemeinsamen Destillationskolonne durchführt.

Dabei erfolgt in einem Teilbereich der Trennwandkolonne die Veresterung der Keto-L-gulonsäure bzw. der Diaceton-2-keto-L-gulonsäure, in einem zweiten Teilbereich wird die Umlagerung zum Ascorbinsäuresalz durchgeführt und gleichzeitig die für die Umlagerung erforderliche Base in einem dritten Teilbereich hergestellt.

Ganz besonders bevorzugt ist ein Verfahren zur Herstellung von Natrium-L-ascorbat, dadurch gekennzeichnet, daß man in einer Trennwandkolonne kontinuierlich
a) 2-Keto-L-gulonsäure mit n-Butanol in Gegenwart von Schwefelsäure in einem separaten Teilbereich der Trennwandkolonne bei gleichzeitigem Abdestillieren des Wassers verestert,
b) den gebildeten 2-Keto-L-gulonsäure-n-butylester in einem weiteren Teilbereich der Trennwandkolonne in Gegenwart von Natrium-n-butylat zu Natriumascorbat umlagert, wobei man
c) Natrium-n-butylat in einem dritten Teilbereich der Trennwandkolonne in-situ durch Umsetzung von n-Butanol mit einer wäßrigen Natronlauge bei gleichzeitigem Abdestillieren des Wassers herstellt.

Die Veresterung mit n-Butanol im Verfahrensschritt a) erfolgt in einem Temperaturbereich von 80 bis 150°C, in einem Druckbereich von 200 bis 950 mbar und bei einer Verweilzeit von 30 bis 250 Sekunden.

Die Umlagerung zu Natrium-L-ascorbat im Verfahrensschritt b) erfolgt in einem Temperaturbereich von 80 bis 120°C, in einem Druckbereich von 200 bis 950 mbar und bei einer Verweilzeit von 40 Sekunden bis 3 Minuten.

Die Herstellung des Natrium-n-butylats im Verfahrensschritt c) erfolgt in einem Temperaturbereich von 80 bis 150°C, in einem Druckbereich von 200 bis 950 mbar und bei einer Verweilzeit von 30 bis 250 Sekunden.

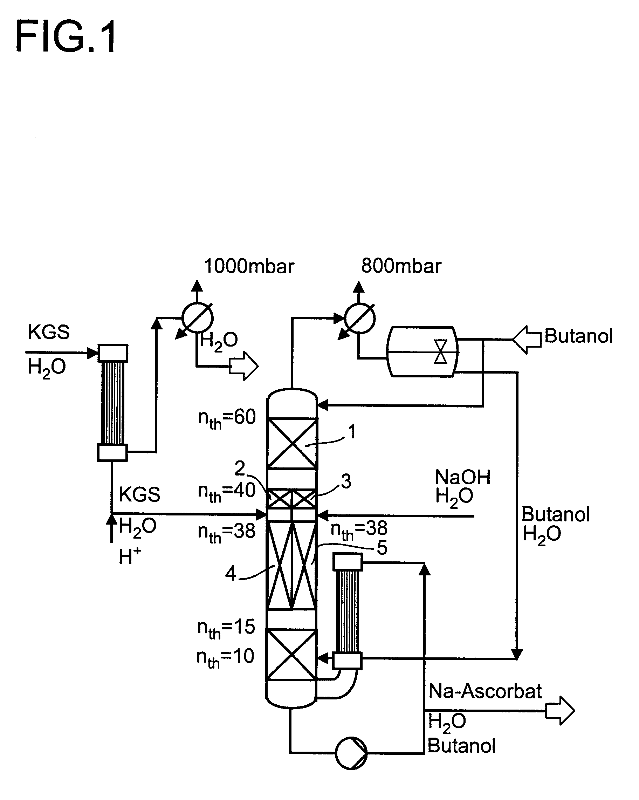

Anhand von Abb. 1 läßt sich die für das erfindungsgemäße Verfahren bevorzugte Apparatur - die sogenannte Trennwandkolonne - am Beispiel von Natrium-L-ascorbat aus 2-Keto-L-gulonsäure näher beschreiben.

In der Trennwandkolonne wird die wäßrige oder alkoholische Lösung von 2-Keto-L-gulonsäure (KGS) im Teilbereich 4 zunächst weiter aufkonzentriert und im gleichen Teilbereich 4 durch Reaktion mit dem Alkohol, vorzugsweise Butanol in Gegenwart der Katalysatorsäure, vorzugsweise Schwefelsäure oder einem sauren Trägerkatalysator, verestert. Der Ester verläßt den Teilbereich 4 als alkoholische Lösung.

Für die Darstellung des Keto-L-gulonsäureesters in der Reaktionskolonne wird bevorzugt eine wäßrige Lösung der freien Säure in einem Konzentrationsbereich von 10 bis 60 Gew.-% eingesetzt.

In einem anderen Segment der geteilten Kolonne erfolgt in dem Teilbereich 5 die Herstellung von wasserfreiem Alkalimetallalkoholat, vorzugsweise Natriumbutylat durch Zufuhr von wässriger Natronlauge und Reaktion mit dem gleichzeitig zudosierten Butanol. Das am Ende des Teilbereiches 5 gebildete Alkalialkoholat wird in das Bett 6 überführt. Dort erfolgt die weitere Umlagerung des Esters zum Alkalimetallascorbat, insbesondere Natrium-L-ascorbat. Die Reaktionsbedingungen und Dimensionen des Teilbereiches 6 werden dabei so abgestimmt, daß das entstandene Alkalimetallascorbat erst im unteren Teil der Packung ausfällt und als Suspension die Kolonne verläßt.

Die Restentwässerung der Kopfprodukte aus der Veresterung und Alkoholatbildung wird im Teilbereich 1 der Trennblechkolonne durchgeführt. Bei Verwendung von Butanol wird das Azeotrop Wasser/n-Butanol am Kopf der Kolonne entnommen und über einen Phasenabscheider geleitet. Das feuchte n-Butanol wird wieder zum Kolonnenkopf zurückgeführt. Zusätzlich kann an dieser Stelle Frischbutanol eingespeist werden.

Anstelle von Wasser eignen sich auch andere polare Solventien, vorzugsweise Alkohole oder Lösungsmittelgemische, um eine Lösung der 2-Keto-L-gulonsäure für die Reaktivdestillation herzustellen.

Für den Einbau heterogener Katalysatoren in Destillationskolonnen sind in der Literatur zahlreiche Konstruktionsmöglichkeiten beschrieben. Hierzu gehören Verweilzeitböden, bei denen der Katalysator auf den Böden oder in deren Ablaufschächten angeordnet sein kann, ferner beschichtete Füllkörper, gewickelte und strukturierte Packungen mit eingewebtem Katalysator. Dafür kommen ebenfalls beliebige saure Katalysatoren, wie z.B Ionentauscher, Zeolithe oder mit sauren Zentren dotierte inerte Träger, in Frage.

Der Ort der Zudosierung der flüssig-homogenen sauren Katalysatoren in die Reaktionskolonne ist von den Betriebsparametern abhängig. Sie variiert vom gleichzeitigen Zusatz mit dem Alkohol am Kopf der Kolonne bis hin zu einer separaten Dosierstelle im Abtriebsteil. Die Art der Zudosierung variiert mit der Reaktionstemperatur, dem Betriebsdruck und der Temperaturbeständigkeit der Katalysatoren. Eine höhere Reaktionsgeschwindigkeit erlaubt dabei eine kompaktere Gestaltung des Apparates.

Die eingangs genannten Temperaturbereiche sind so zu wählen, daß der Katalysator, die Einsatzstoffe und das Reaktionsprodukt nicht geschädigt werden. Gleichzeitig ist es wünschenswert, die Kondensationstemperaturen deutlich über der Umgebungstemperatur zu halten.

Zur Herstellung des Alkalimetallalkoholates werden die gleichen Temperatur- und Druckbereiche angewendet, wie sie sich zur Herstellung des Esters ergeben. Im Falle des Butylats arbeitet man unter Normal- oder schwachem Unterdruck im Temperaturbereich von 100 bis 130°C.

Es ist außerdem möglich, daß man Verfahrensschritt b) in einem von der Destillationskolonne abgetrennten Reaktor durchführt.

Ein solches, ebenfalls bevorzugtes Verfahren ist dadurch gekennzeichnet, daß man in einer Trennwandkolonne kontinuierlich
a) 2-Keto-L-gulonsäure mit n-Butanol in Gegenwart von Schwefelsäure in einem separaten Teilbereich der Trennwandkolonne bei gleichzeitigem Abdestillieren des Wassers verestert,
b) den gebildeten 2-Keto-L-gulonsäure-n-butylester in einer von der Trennwandkolonne abgetrennten Reaktionsmischpumpe in Gegenwart von Natrium-n-butylat kontinuierlich zu Natriumascorbat umlagert, wobei man
c) Natrium-n-butylat in einem weiteren separaten Teilbereich der unter a) genannten Trennwandkolonne in-situ durch Umsetzung von n-Butanol mit einer wäßrigen Natronlauge bei gleichzeitigem Abdestillieren des Wassers herstellt.

Die Druck-, Temperatur- und Verweilzeitparameter der einzelnen Stufen a) bis c) liegen auch hier in den bereits oben genannten Bereichen.

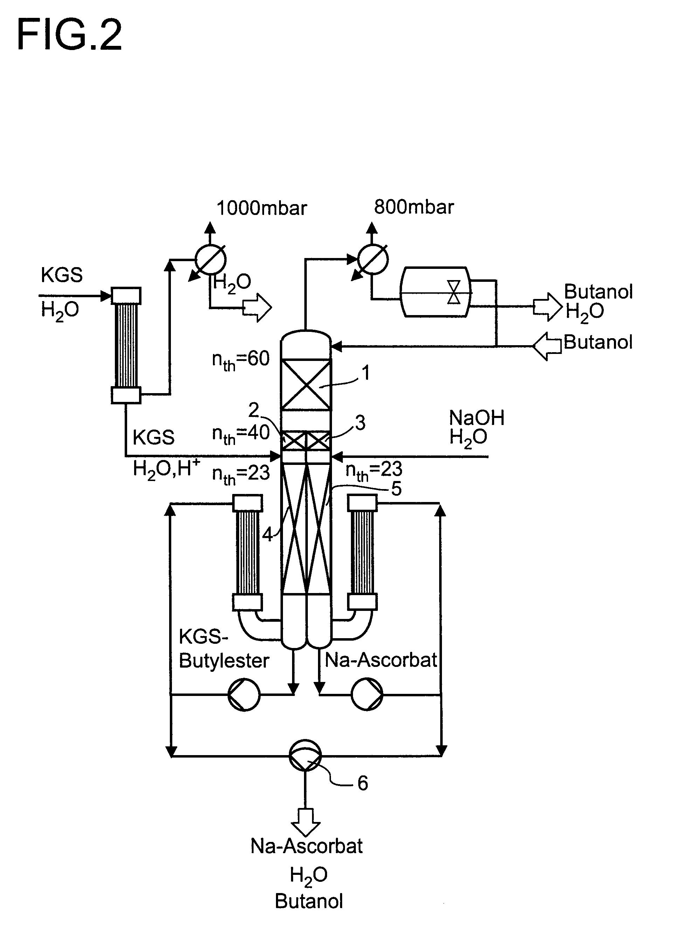

So können, entsprechend der Abbildung 2, die Produktströme des 2-Keto-L-gulonsäureesters und des Alkalialkoholats separat der Trennblechkolonne entnommen werden, anschließend in einer Reaktionsmischpumpe (6) vereinigt und in Alkalialscorbat überführt werden und die gegebenenfalls entstehende Suspension einer nachfolgenden Aufreinigungsstufe zugeführt werden. Alternativ zur Reaktionsmischpumpe können auch Zylinderwalzen oder Strahldüsenreaktoren für den Lactonisierungsschritt verwendet werden.

Ebenso läßt sich das C₁-C₁₀-Alkalialkoholat in separaten Reaktoren herstellen, um dann anschließend kontinuierlich durch Zuführung in einen der oben beschriebenen Reaktoren die Umlagerung des 2-Keto-L-gulonsäureesters zu katalysieren.

Das erfindungsgemäße kontinuierliche Verfahren zur Herstellung von Alkalisalzen der L-Ascorbinsäure zeichnet sich durch eine Reihe von Vorteilen aus:
- Die Gesamt-Verweilzeiten sind im Vergleich zu den literaturbekannten Ausführungsformen der Ascorbat-Synthesen ausgehend von 2-Keto-L-gulonsäure sehr kurz.
- Dadurch können Apparate kleiner Dimensionen eingesetzt werden.
- Die Gleichgewichtsumsätze bei der Veresterung sind hoch, wodurch das Reaktionsprodukt je nach spezifischen intensiven Reaktionsparametern einen Restgehalt an nicht umgesetzter 2-Keto-L-gulonsäure von nur 0,02 bis 0,5 Gew.-% aufweist.
- Die Arbeitsbedingungen während der Abtriebsdestillation können so eingestellt werden, daß der Wassergehalt und ggf. auch der Anteil des Veresterungsalkohols im Reaktionsgemisch gegen Null gehen.
- Die kontinuierliche Alkoholatherstellung kann unter vollständiger Entwässerung in derselben Destillationskolonne erfolgen.
- In der Kolonne erfolgt die Wasserauskreisung und die Butanolrückgewinnung.
- Die Restentwässerung der Keto-L-gulonsäurelösung erfolgt ebenfalls im selben Apparat.

Anhand des folgenden Beispiels soll das erfindungsgemäße Verfahren näher erläutert werden.

### Beispiel 1

### Herstellung von Natrium-L-ascorbat

Die kontinuierliche Herstellung von 2-Keto-L-gulonsäure-n-butyl-ester und Natrium-n-butylat sowie die anschließende Umlagerung zu Natrium-L-ascorbat wurden in einer Trennwandkolonne gemäß Abbildung 1 durchgeführt.

Die Versuchskolonne hatte einen Durchmesser von 0,2 m. Die Kolonne war über einer Höhe von 20 m mit Blechpackungen mit einer spezifischen Oberfläche von 350 m²/m³ bestückt. Die theoretische Trennstufenzahl der Kolonne lag bei insgesamt 60 Trennstufen. Die Trennwand der Kolonne war zwischen der 15. und der 40. Stufe (von unten her gezählt) durch Einschweißung angebracht. Die Aufteilung der Flüssigkeit auf die Teilbereiche 2 und 3 der Kolonne erfolgte im Mengenverhältnis von 2 : 1. Die Kolonne wurde bei einem Kopfdruck von 800 mbar betrieben. Der Zulauf auf der Veresterungsseite der Trennblechkolonne (Teilbereiche 2 und 4) betrug 62,5 kg/h in Form einer 50 Gew.-%igen, wäßrigen 2-Keto-L-gulonsäure-Lösung. Die als Katalysator eingesetzte Schwefelsäure wurde als 5 Gew.-%ige butanolische Lösung mit einer Geschwindigkeit von 7,25 kg/h zudosiert.

Auf der Alkoholatseite der Trennblechkolonne (Teilbereiche 3 und 5) wurden 128 kg/h 50% Natronlauge zugefahren.

Das im Verstärkungsteil 1 anfallende Azeotrop n-Butanol/Wasser wurde am Kopf der Kolonne kondensiert und einem Phasentrenngefäß zugeführt. Die Butanolphase wurde auf den Kopf der Kolonne zurückgefahren. Die wäßrige Phase wurde in den Abtriebsteil der Kolonne am 10ten theoretischen Boden (von unten her gezählt) eingeleitet.

In einer geeigneten Packung 6 wurden die Ester- und Alkoholat-Produktströme zusammengeführt und zügig aus der Kolonne herausgefahren. Die schwach gelb gefärbte Suspension enthielt Natrium-L-ascorbat. Nach dem Abfiltrieren, Waschen mit n-Butanol und Trocknen im Vakuum wurde rohes Natrium-L-ascorbat mit einer Reinheit von 98% in einer Ausbeute von 94% isoliert.

## Patentansprüche

1. Verfahren zur Herstellung von Alkalisalzen der L-Ascorbinsäure, enthaltend die folgenden Schritte:
a) Veresterung von 2-Keto-L-gulonsäure oder Diaceton-2-keto-L-gulonsäure mit einem C₁-C₁₀-Alkohol in Gegenwart eines sauren Katalysators,
b) Umlagerung des gebildeten 2-Keto-L-gulonsäure-C₁-C₁₀-al-kylesters in Gegenwart eines C₁-C₁₀-Alkalialkoholats,
dadurch gekennzeichnet, daß man die Verfahrensschritte a) und b) jeweils kontinuierlich durchführt.

2. Verfahren nach Anspruch 1, dadurch gekennzeichnet, daß man im Verfahrensschritt a) die Veresterung mit einem C₃-C₈-Alkohol, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol durchführt.

3. Verfahren nach einem der Ansprüche 1 oder 2, dadurch gekennzeichnet, daß man die Veresterung in Gegenwart eines sauren Katalysators ausgewählt aus der Gruppe, bestehend aus Mineralsäuren, organischen Sulfonsäuren, organischen Carbonsäuren, sauren Ionenaustauscherharzen und Festbettkatalysatoren mit sauren Reaktionszentren durchführt.

4. Verfahren nach einem der Ansprüche 1 bis 3, dadurch gekennzeichnet, daß man im Verfahrensschritt b) die Umlagerung in Gegenwart eines Alkalialkoholats eines C₃-C₈-Alkohols, ausgewählt aus der Gruppe, bestehend aus n-Propanol, Isopropanol, 1-Butanol, 2-Butanol, 2-Methyl-1-propanol, 2-Methyl-2-propanol, 1-Pentanol, 1-Hexanol und 1-Octanol durchführt.

5. Verfahren nach einem der Ansprüche 1 bis 4, dadurch gekennzeichnet, daß man in einem zusätzlichen Verfahrensschritt c) das C₁-C₁₀-Alkalialkoholat in-situ erzeugt.

6. Verfahren nach Anspruch 5, dadurch gekennzeichnet, daß man die Herstellung des Alkalialkoholates im Verfahrensschritt c) in einem Temperaturbereich von 40 bis 250°C, in einem Druckbereich von 1 bis 4000 mbar und bei einer Verweilzeit von 1 bis 2000 Sekunden durchführt.

7. Verfahren nach einem der Ansprüche 1 bis 6, dadurch gekennzeichnet, daß man die Veresterung im Verfahrensschritt a) in einem Temperaturbereich von 40 bis 250°C, in einem Druckbereich von 1 bis 2000 mbar und bei einer Verweilzeit von 1 bis 2000 Sekunden durchführt.

8. Verfahren nach einem der Ansprüche 1 bis 7, dadurch gekennzeichnet, daß man die Umlagerung im Verfahrensschritt b) in einem Temperaturbereich von 40 bis 200°C, in einem Druckbereich von 1 bis 4000 mbar und bei einer Verweilzeit von 20 Sekunden bis 60 Minuten durchführt.

9. Verfahren nach Anspruch 5, dadurch gekennzeichnet, daß man mindestens einen der Verfahrensschritte a) bis c) in einer Destillationskolonne durchführt.

10. Verfahren nach Anspruch 9, dadurch gekennzeichnet, daß es sich bei der Destillationskolonne um eine Trennwandkolonne handelt.

11. Verfahren nach einem der Ansprüche 9 oder 10, dadurch gekennzeichnet, daß man alle drei Verfahrensschritte a) bis c) in einer gemeinsamen Destillationskolonne durchführt.

12. Verfahren nach einem der Ansprüche 9 oder 10, dadurch gekennzeichnet, daß man Verfahrensschritt b) in einem von der Destillationskolonne abgetrennten Reaktor durchführt.

13. Verfahren nach Anspruch 11, dadurch gekennzeichnet, daß man in einer Trennwandkolonne kontinuierlich
a) 2-Keto-L-gulonsäure mit n-Butanol in Gegenwart von Schwefelsäure in einem separaten Teilbereich der Trennwandkolonne bei gleichzeitigem Abdestillieren des Wassers verestert,
b) den gebildeten 2-Keto-L-gulonsäure-n-butylester in einem weiteren Teilbereich der Trennwandkolonne in Gegenwart von Natrium-n-butylat zu Natriumascorbat umlagert, wobei man
c) Natrium-n-butylat in einem dritten Teilbereich der Trennwandkolonne in-situ durch Umsetzung von n-Butanol mit einer wäßrigen Natronlauge bei gleichzeitigem Abdestillieren des Wassers herstellt.

14. Verfahren nach Anspruch 12, dadurch gekennzeichnet, daß man in einer Trennwandkolonne kontinuierlich
a) 2-Keto-L-gulonsäure mit n-Butanol in Gegenwart von Schwefelsäure in einem separaten Teilbereich der Trennwandkolonne bei gleichzeitigem Abdestillieren des Wassers verestert,
b) den gebildeten 2-Keto-L-gulonsäure-n-butylester in einer von der Trennwandkolonne abgetrennten Reaktionsmischpumpe in Gegenwart von Natrium-n-butylat kontinuierlich zu Natriumascorbat umlagert, wobei man
c) Natrium-n-butylat in einem weiteren separaten Teilbereich der unter a) genannten Trennwandkolonne in-situ durch Umsetzung von n-Butanol mit einer wäßrigen Natronlauge bei gleichzeitigem Abdestillieren des Wassers herstellt.

15. Verfahren nach einem der Ansprüche 13 oder 14, dadurch gekennzeichnet, daß man die Veresterung im Verfahrensschritt a) in einem Temperaturbereich von 80 bis 150°C, in einem Druckbereich von 200 bis 950 mbar und bei einer Verweilzeit von 30 bis 250 Sekunden durchführt.

16. Verfahren nach einem der Ansprüche 13 bis 15, dadurch gekennzeichnet, daß man die Umlagerung im Verfahrensschritt b) in einem Temperaturbereich von 80 bis 120°C, in einem Druckbereich von 200 bis 950 mbar und bei einer Verweilzeit von 40 Sekunden bis 3 Minuten durchführt.

17. Verfahren nach einem der Ansprüche 13 bis 16, dadurch gekennzeichnet, daß man die Herstellung des Natrium-n-butylats im Verfahrensschritt c) in einem Temperaturbereich von 80 bis 150°C, in einem Druckbereich von 200 bis 950 mbar und bei einer Verweilzeit von 30 bis 250 Sekunden durchführt.
